(19) Europäisches Patentamt
European Patent Office
Office européen des brevets

(11) **EP 3 502 694 A1**

(12) **EUROPEAN PATENT APPLICATION**

(43) Date of publication:
**26.06.2019  Bulletin 2019/26**

(51) Int Cl.:
***G01N 33/52*** *(2006.01)*

(21) Application number: **18195469.4**

(22) Date of filing: **19.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.12.2017  EP 17209747**

(71) Applicant: **PAUL SCHERRER INSTITUT
5232 Villigen PSI (CH)**

(72) Inventors:
• **AEPPLI, Gabriel
5210 Windisch (CH)**

• **BAILEY, Joe
8004 Zürich (CH)**
• **DALBY, Paul
London NW1 9HG (GB)**
• **HALES, John
London SE13 6UJ (GB)**
• **MATMON, Guy
London NW1 1BA (GB)**

(74) Representative: **Fischer, Michael
Siemens AG
Postfach 22 16 34
80506 München (DE)**

(54) **METHOD FOR BIO-SENSING A BINDING ABILITY AMONG A BIOMOLECULE AND A VIRUS USING VIRAL-LASING DETECTION PROBES**

(57)    The selective amplification of DNA in PCR is used to exponentially increase the signal in molecular diagnostics for nucleic acids, but there are no analogous techniques for signal enhancement in clinical tests for proteins or cells. Instead, the signal from affinity-based measurements of these biomolecules depends linearly on the probe concentration. Substituting antibody-based probes tagged for fluorescent quantification with lasing detection probes would enable a new platform for biomarker quantification based on optical amplification. Here, we construct a viral laser which bridges synthetic biology and laser physics, and demonstrate viral-lasing probes for bio-sensing. At the transition to lasing, the photon flux from the probes increases by five orders of magnitude and narrows the spectral linewidth to below 5.0 nm. Viral-lasing probes display an unprecedented > 500,000 % increase in signal from only a 50 % increase in probe concentration, using fluorimeter-compatible optics, and can detect biomolecules at clinically-relevant concentrations.

Figure 5

EP 3 502 694 A1

**Description**

The present invention relates to a method for bio-sensing a binding ability among a biomolecule, such as an antibody, a membrane protein or the like, and a virus.

Antibody-based probes tagged for fluorescent quantification can bind a broad range of biomolecular targets with high specificity, but generate a weak signal which can be difficult to distinguish from background noise. Lasing detection probes would be a superior alternative, as they would generate intense, monochromatic signals and the onset of lasing would coincide with a sharp increase in the radiant energy in a resonator-dependent emission line. However, no new detection probes have been developed which can both harness lasing and also bind a broad range of epitopes with the specificity of antibodies, despite the construction of lasers from biological components, such as DNA scaffolds and fluorescent-protein-expressing cells. Dye-labelled antibodies themselves are not ideal candidates because they can only be conjugated with up to four dyes per antibody before the degree of labelling interferes with target binding and the yield of the dyes, and their size and structure limit the scope for genetic engineering and chemical modification.

Filamentous bacteriophage M13 - a 7 nm x 900 nm rod-like virus that infects F pilus expressing strains of *E. coli* - has been used effectively as a detection probe for cell imaging, flow cytometry and ELISA and as the key component in nanotechnologies, including virus-based lithium ion batteries and piezoelectric generators. They can be routinely programmed to bind specific target biomolecules either by display of known antibody domains or binding proteins fused to either the gene 3 or 8 coat proteins, or by selection from phage-displayed combinatorial libraries to isolate those with the required target-binding specificity and affinity.

It is therefore the objective of the present invention to provide a method for micro-biological probes that delivers a significantly higher signal to noise ratio in molecular binding assays as compared to the classical dye-based fluorescence probes.

This objective is achieved according to the present invention by a method for bio-sensing a binding ability among a biomolecule, such as an antibody, a membrane protein or the like, and a virus, comprising the steps of:

a) labelling the virus by conjugating a plurality of dyes, preferably fluorescein dyes, to the virus in order to achieve a labelled virus being controllable with respect to the density of labelled dyes, preferably fluorescein dyes, per virus;
b) binding the labelled virus to the biomolecule in a liquid solution to the extent the binding characteristics among the biomolecule and the labelled virus allow for in a ligand binding assay in order to generate biomolecule virus compounds;
c) pumping the liquid solution comprising the biomolecule virus compounds with light having a wavelength and/or an intensity being adopted to the type of the effective mechanism of the dyes, preferably the fluorescence mechanism of the fluorescein dyes, labelled to the virus;
d) amplifying the light emitted from the pumped dyes in an optical resonator in order to achieve a lasing response of the excited dyes; and
e) measuring the intensity of the amplified emitted light and evaluating the intensity of the amplified emitted light against the known density of labelled fluorescein dyes per virus molecule or against an equivalent concentration of the same number of dyes per probe in order determine the binding ability of the biomolecule to the labelled virus.

This method therefore represents a viral-lasing detection mechanism which can bind the full range of biomolecules targeted in clinical assays, including proteins, nucleic acids and cells, whilst also generating a lasing signal in an optical configuration compatible with ordinary fluorimeters. The structural order and repeating chemical landscape on the surface of viruses modulate the spectroscopy and threshold dynamics of the laser, providing an amenable and versatile model system to study lasing using the principles of synthetic biology. At the transition to lasing, the photon flux from the conjugated viruses increases by five orders of magnitude, the spectral linewidth narrows to below 5.0 nm, and the sensitivity of the output to small changes in the probe concentration or environment is heightened.

Preferred embodiments of the present invention are listed hereinafter and can be applied and can be applied alone or in arbitrary combinations:

a) the virus is a M13 bacteriophage or a recombinant Tobacco mosaic virus like particle (rTMV);

b) the biomolecule can be any molecule that the virus is engineered to bind, for examplean IgG2a monoclonal antibody (mAB);

c) the fluorescein dye is a fluorescein isothiocyanate isomer 1 dye;

d) the virus is covalently modified with the fluorescein dye;

e) the pumping is achieved using 1 ns to 20 ns excitation pulses in the wavelength of the visible light, preferably in the range of 450 to 600 nm;

f) the liquid solution is circulated between a reservoir and a flow cuvette;

g) the virus is effectively genetically-programmable with a spectral peak emission that is tunable by varying the number of fluorescein dyes attached per virus and/or by modifying the chemical landscape of the surface of the virus; and/or

h) the virus is conjugated with a number of fluorescein dyes equivalent to a range of 0.5 to 2 dyes, preferably 0.8 to 1.2 dyes, on average per ring of coat proteins of the virus.

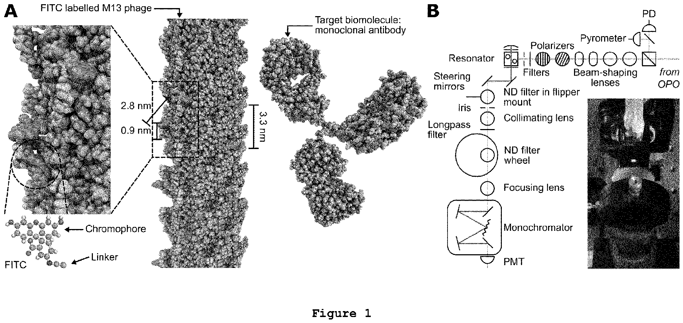

In particular, the surface of the M13 bacteriophage can be functionalised with amine-reactive dyes that could attach to either the $\alpha$-amines at Ala1 or the $\epsilon$-amines at Lys8 on the 2700 50 amino-acid alpha-helical gene 8 coat proteins or the solvent-exposed primary amines on the five gene 3 coat proteins. The gene 8 coat proteins form an overlapping quasi-crystalline lattice with a 5-fold rotation axis and a two-fold screw axis, which brings the dyes into close proximity - much smaller than the wavelength of visible light - enabling electronic interactions and leading to resonant energy transfer between dye molecules as well as fluorescence quenching (see Fig. 1a).

Other advantageous features of the present invention are listed in the depending claims.

Preferred embodiments of the present invention are hereinafter described in more detail with respect to the attached drawings which depict in:

Figure 1   schematically the design of lasing detection probe composed of M13 bacteriophage (PDB: 2MJZ21) covalently modified with fluorescein isothiocyanate isomer 1 dyes;

Figure 2   a series of threshold curves for different probe concentrations;

Figure 3   coupling viral-lasing to interactions between neighbouring dyes and their environment;

Figure 4   the response of the viral laser to ligand-binding; and

Figure 5   schematically an optical configuration for experiments conducted using a, resonant cavity R1 and b, resonant cavity R2.

As one representative example, the present invention provides a viral laser from fluorescein-dye-labelled M13 and demonstrates its capabilities as a new analytical platform for biomedicine. Viral-lasing detection probes are the first probes which can bind the full range of biomolecules targeted in clinical assays, including proteins, nucleic acids and cells, whilst also generating a lasing signal in an optical configuration compatible with ordinary fluorimeters. The structural order and repeating chemical landscape on the surface of M13 modulate the spectroscopy and threshold dynamics of the laser, providing an amenable and versatile model system to study lasing using the principles of synthetic biology. At the transition to lasing, the photon flux from the probes increases by five orders of magnitude, the spectral linewidth narrows to below 5.0 nm, and the sensitivity of the output to small changes in the probe concentration or environment is heightened. It is shown that a 50 % increase in the concentration of the probes from 100 pmol/mL results in a > 500,000 % increase in signal, which is the greatest responsivity to probe concentration shown in any biological assay to the best of our knowledge.

In a proof-of-concept study, a mix-and-measure ligand-binding assay sensitive to 90 fmol/mL monoclonal antibody has been optically engineered, suggesting that clinically-relevant concentrations of biomolecules can be detected without immobilisation of the ligand or probe on surfaces and without invasive wash steps.

Figure 1 shows schematically the concept of viral lasers. Part a) shows a model of the atomic structure of a lasing detection probe composed of M13 bacteriophage (PDB: 2MJZ) covalently modified with fluorescein isothiocyanate isomer 1 dyes (PubChem CID: 18730). There are approximately 540 rings of major coat proteins per M13, but only 13 are shown here. The target biomolecule is a IgG2a monoclonal antibody (mAb) that can be bound by the gene 3 coat proteins and is represented in the figure by the structure of an intact IgG2a mAb (PDB: 1IGT) which should have a homologous structure. Inset, a zoomed image of the surface of M13 shows the close proximity of the attached dyes. Further inset, chemical structure of fluorescein isothiocyanate is depicted. Part b) illustrates schematically the optical setup of the viral laser used to construct a mix-and-measure ligand-binding assay. Inset, a photograph of the viral laser emission as viewed from along the axis of the resonator is shown.

For the design of the viral lasing, the properties of viral lasers have been investigated in which the gain medium is a solution of fluorescein-dye-labelled M13 in two contrasting resonator geometries. Resonant cavity R1 was constructed to explore viral-lasing for metrology, and resonant cavity R2 was engineered to strengthen the coupling of the viral-lasing to the near-field and electronic interactions of the dyes (as shown in Fig. 1b). In both instruments the excitation and detection optics were similar to those in a fluorimeter, except for the greater than eight-decade dynamic range of the spectrometer and the intensity of the 3 ns to 5 ns excitation pulses at 493 nm (see Fig. 1b, details are described in the section Methods hereinafter). The gain medium was circulated between a reservoir and a flow cuvette and the pump-light source operated in single-shot mode to allow the dye to be refreshed between pulses as shown in Figure 1b.

The resonator R1 comprises a flat mirror separated by 300 mm from a spherical mirror with a radius of curvature of 400 mm, equivalent to 2 round-trips per FWHM (Full Width at Half Maximum) of the temporal profile of the pump laser pulse. The diameter of the beam was constrained only by the 2 mm x 2 mm windows of the cuvette. In contrast to micro-resonators, these dimensions are much greater than microbiological length scales ensuring that there was no mechanical interference from the resonator.

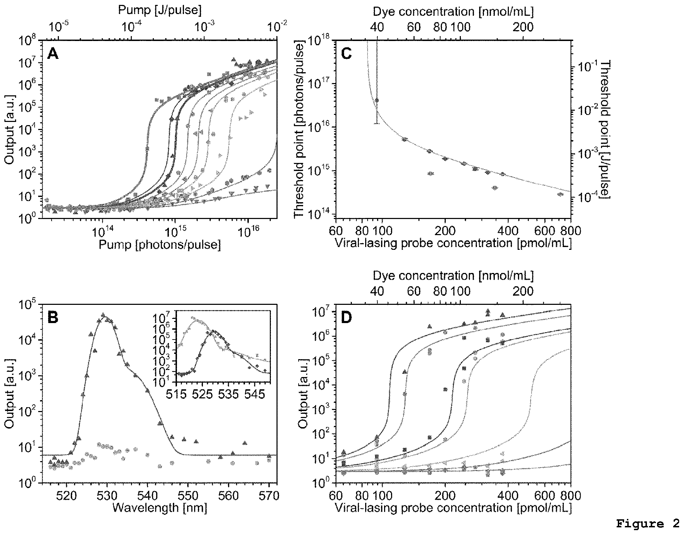

Figure 2 shows lasing action for the functionalised virus and demonstrates its strong sensitivity to the probe concentration in the buffer solution. In detail, Figure 2 shows viral lasers and quantification of viral-lasing probes. (A) Threshold behaviour in R1 for 146 nmol/mL fluorescein (F1, dark orange) and for viral-lasing probes (V1) diluted from 377 pmol/mL (purple) to 320 pmol/mL (dark blue, equivalent to 135 nmol/mL fluorescein), 246 pmol/mL (green), 200 pmol/mL (light purple ), 169 pmol/mL (cyan), 128 pmol/mL (light green), 94 pmol/mL (blue), and 65 pmol/mL (grey). The fitted curves represent a global fit of all of the sets of threshold data to a theoretical model. (B) Emission spectra of V1 at 377 pmol/mL as pumping increased from $5.5 \times 10^{14}$ photons/pulse $\pm$ 10.1 % (cyan) to $9.1 \times 10^{14}$ photons/pulse $\pm$ 6.6 % (dark blue). Inset, bathochromic shift in the spectrum of V1 (purple, pumping of $1.5 \times 10^{15}$ photons/pulse $\pm$ 10.9 %) relative to 72 nmol/mL fluorescein (orange, $1.7 \times 10^{16}$ photons/pulse $\pm$ 11.4 %). Curves are fits to the sum of two Gaussian peak functions. The output was not calibrated but was proportional to the number of photons entering the spectrometer (see Methods). (C) Response of the threshold point to variations in probe concentration. Scatter points represent the threshold point calculated by fitting individual sets of threshold data of either V1 (dark blue) or fluorescein (dark orange) using a simple function. The line is the expected dependence of the threshold point on the probe concentration calculated from the global fit in (A). (D) The output at different probe concentrations has been plotted at pump energies of

$1.0 \times 10^{16}$ photons/pulse $\pm$ 5.7 % (dark blue),

$5.4 \times 10^{15}$ photons/pulse $\pm$ 7.6 % (green),

$1.8 \times 10^{15}$ photons/pulse $\pm$ 6.2 % (purple),

$1.4 \times 10^{15}$ photons/pulse $\pm$ 5.3 % (cyan),

$5.5 \times 10^{14}$ photons/pulse $\pm$ 4.8 % (light green),

$2.5 \times 10^{14}$ photons/pulse $\pm$ 6.3 % (blue),

$9.6 \times 10^{13}$ photons/pulse $\pm$ 5.8 % (light purple). The lines are simulations from the global fit (see a) for the output against probe concentration at the same pump energies.

In Fig. 2a, a series of threshold curves for different probe concentrations is displayed as well as for fluorescein in solution with no virus. For the highest probe concentration (purple), 377 pmol/mL M13 conjugated with 422 dyes per M13 (V1) - equivalent to 0.8 dyes on average per ring of proteins - increasing the pump energy by a factor of 2.0 from below to above the threshold point for lasing, increased the output energy at the spectral peak by a factor of 16,000 (Fig. 2a).

The threshold region of the viral laser has been measured with unprecedented precision across 7 decades of output because it is this non-linearity that makes lasing such a unique analytical prospect. This contrasts attempts at similar measurements for other bio lasers, which have typically been restricted to 1 to 2 decades of dynamic range. Moreover, if only the above-threshold region is measured, then the output increases linearly with pumping and the position of the threshold point can only be extrapolated, reducing the potential utility of these bio lasers as analytical instruments. In Fig. 2a, the initial deviations in output from sub-threshold behaviour are the build-ups in oscillation due to the amplification of seed fluorescence before the gain can overcome the resonator losses, and the inflection points are the transitions to lasing.

The threshold for 146 nmol/mL fluorescein (F1, dark orange) was 2.2 times lower than for 320 pmol/mL V1 (dark blue), which had a similar volume-averaged optical density equivalent to 135 nmol/mL fluorescein. This implies that there was enhanced quenching of the dyes attached to M13 due to dye-dye or dye-protein interactions, since the lasing threshold $\varphi_{th}$ varies according to,

$$(1)\ \varphi_{th} = \frac{K_2\, K_L}{K_F\, (D\, K_{RAD} - K_L)}$$

The microenvironments provided by the virus and solvent for the dyes will affect the decay rate $K_2$ from the upper state of the dye, but optical absorption data show no influence on the strength $K_F$ of the coupling of the pump to the dyes. If the resonator, defined by the loss rate $K_L$, the rate $K_{RAD}$ of spontaneous emission per dye into the resonator mode and the number D of dyes in the mode volume are unchanged and D is large, the ratio of ultimate lasing output $n$ to pump power $\varphi$ will be the same for V1 and F1 because in the high power limit,

$$(2) \quad \frac{n}{\varphi} \rightarrow \frac{\beta\,K_F}{K_{RAD}}\left(\frac{D\,K_{RAD}}{K_L} - 1\right) \rightarrow \frac{\beta\,K_F\,D}{K_L}$$

where $\beta$ is the optical attenuation due to the output coupler and the spectral bandwidth of the spectrometer (equations derived in Theoretical Models). The data in Fig. 2a are consistent with eq. (2).

The curves in Fig. 2a have been derived from a model of the threshold dynamics that describes the energy build-up in the dominant mode in terms of the pump energy and the dye concentration while accounting for the seed fluorescence. The data has been globally fit with shared parameters for experimental variables retained between measurements, such as the number of resonator modes and fixed values for variables which could be calculated in advance, including the rate of loss from the resonator. From this model and by noting that from eq. (1) the threshold point is proportional to $\left(\frac{D}{D_{min}} - 1\right)^{-1}$, it has been estimated that the minimum number of probes required for lasing is

$$D_{min} = \frac{K_L}{K_{RAD}} = 84.6 \text{ pmol/mL} \pm 0.8 \text{ pmol/mL},$$

which is three orders of magnitude lower than the lowest reported DNA FRET laser probe concentration. This value is at the upper limit of the useful range for probes in a clinical context, but it could be decreased straightforwardly by minimising Fresnel reflections at the cuvette side-walls, or by reducing the number of cavity modes with mode selective optical elements or by shortening the resonant cavity.

Spectral linewidth narrowing has not been shown for a solution-state bio laser previously and even here the spectral measurement below threshold has been hampered by low light levels. Even so, Fig. 2b demonstrates that the sharp rise in output at threshold coincided with a change in the spectral line-shape and a narrowing of the spectral linewidth to 4.0 nm $\pm$ 0.3 nm (FWHM) above threshold at a pump energy of $9.1 \times 10^{14}$ photons/pulse $\pm$ 6.6 %.

Unlike for a DNA FRET laser or a GFP cell laser, the biological structural order of M13 impacts the spectroscopic characteristics of a homogeneous ensemble of chromophores in the viral laser. Moving the dyes closer together first by increasing the density in solution and then via immobilization on the viruses red-shifted the spectral peak from 523.4 nm $\pm$ 0.2 nm for 72 nmol/mL fluorescein (mean separation 28.5 nm) to 527.2 nm $\pm$ 0.4 nm for 301 nmol/mL fluorescein (17.7 nm) to 529.6 nm $\pm$ 0.2 nm for V1 (Fig. 2b inset). Thus, lasing viruses are effectively genetically-programmable quantum dots with a spectral peak emission that can be tuned by varying the number of dyes attached per virus, or by modifying the chemical landscape of the surface of the virus.

Further, the quantification of viral-lasing probes will be discussed. Fig. 2c and Fig. 2d show that the concentration of dye-labelled M13 probes can be determined from the position of the threshold point. A simple function has been derived that can model threshold data without requiring precise knowledge of the experimental variables, and in Fig. 2c this function has been implemented to calculate the threshold points as a function of the probe concentration (dark blue), and in matching units for freely-dissolved fluorescein (dark orange). The data points are in excellent agreement with the cyan curve, which is the expected threshold point position based on the global model of the threshold dynamics (see Fig. 2a), proving that each set of threshold data contained enough information to allow the probe concentration to be calculated precisely.

Typically, threshold data are plotted as the output against pump energy at constant dye concentration, as is the case in Fig. 2a, but in Fig. 2d it is shown that the same data can be plotted as the output against probe concentration at constant pump energy. Mirroring a conventional threshold curve, the output was extremely sensitive to small changes in the probe concentration when the pump energy was close to the associated threshold point. For instance, from the model globally fit to the data, if the pump energy were fixed at $1.001 \times 10^{16}$ photons/pulse, which was the threshold point for 108.6 pmol/mL V1, a 50 % increase in the probe concentration from 100.0 pmol/mL to 150.0 pmol/mL would result in a 650,000 % increase in the output. Away from the threshold point, a 50 % change would simply result in a ~ 50 % change in the output, as is typical for a ratiometric assay, which might be difficult to see above the background. This is the greatest responsivity in output to small changes in probe concentration that has been recorded in any biological assay system, to the best of our knowledge. This analytical concept has immediate applications in tipping point measurements, where the sample would be pumped at the threshold intensity for the critical concentration of lasing probes to yield an unambiguous, digital readout: if there is lasing, then the critical concentration is exceeded, otherwise there is no lasing. Alternatively, the pump energy could be scanned to find the threshold point, and therefore the probe concentration, via cross-referencing with a calibration plot such as Fig. 2c.

Now, coupling viral-lasing with the near-field and electronic interactions of the dyes is discussed. Binding events are likely to disrupt the near-field and electronic interactions occurring at sub-1 ns timescales on the surface of the phage between dyes and between the dyes and their environment, so the resonator (designated R2) length was reduced to 26 mm, corresponding to a round-trip time of 195 ps taking the refractive index of the gain medium into account (Fig. 1b). The number of spatial modes was further reduced by constraining the diameter with a 300 $\mu$m pinhole so that the Fresnel number of the resonator was F = 6.7. Also, the degree-of-labelling was increased to encourage dye-dye interactions.

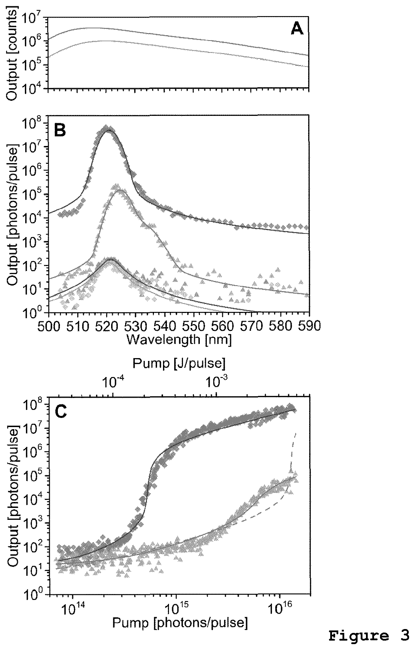

Fig. 3 shows the experimental results for 20.8 pmol/mL M13 conjugated with 618 dyes per M13 (V2), equivalent to 1.1 dyes on average per ring of coat proteins, and for 13.3 nmol/mL of unbound fluorescein dye molecules in buffer (F2). Fig. 3a shows that the fluorescence yield was broad for both V2 and F2, and that V2 (cyan) was shifted by 4 nm to longer wavelengths, and quenched by a factor of 3.5 per dye relative to F2 (dark orange). In the resonant cavity R2, for pump intensities close to but below the threshold for lasing, the emission spectra for both V2 (light purple) and F2 (light orange) were sharpened considerably to peaks very close to each other at 521.4 nm $\pm$ 0.1 nm and 521.0 nm $\pm$ 0.1 nm, with FWHMs of 7.2 nm $\pm$ 0.3 nm and 6.7 nm $\pm$ 0.3 nm, respectively (Fig. 3a, b). This shows that, even below threshold, the interaction between the dyes and the resonant cavity modes is strong enough to narrow the emission peaks compared to free-space fluorescence emission.

As for the more concentrated dye solutions in R1, the transition to lasing led to an increase by many orders of magnitude in the emission intensity at maximum pumping. Above threshold, the V2 emission spectrum (cyan) shows oscillation build-up in two modes - at 524.8 nm $\pm$ 0.16 nm and 530.9 nm $\pm$ 1.4 nm - similar to V1, and compares to just one mode for F2 (dark orange) at 521.1 nm $\pm$ 0.06 nm (Fig. 3d). Therefore, above-threshold pumping caused a red-shift in the V2 emission spectrum which was not observed for F2. The primary V2 peak at 524.8 nm was linewidth-narrowed to 5.7 nm $\pm$ 0.22 nm, which was similar to the FWHM of 5.6 nm $\pm$ 0.08 nm for the single F2 peak. The secondary V2 peak had the same Voigt profile as the primary peak but was broader, with a FWHM of 9.6 nm $\pm$ 0.6 nm. The threshold curves displayed in Fig. 3c revealed other differences between V2 (cyan) and F2 (dark orange). Firstly, the lasing threshold of V2 was increased by a factor of 11.2, which was larger than for V1 relative to F1 because of greater non-radiative losses due to the proximity of the dyes, and was consistent with the reduction in fluorescence yield and eq. (1). Secondly, the output above threshold was severely diminished and the approach to saturation was considerably broadened for V2 compared to F2, which was a qualitatively different outcome than for V1 and F1.

The second observation cannot be readily understood in terms of the rate equations used to model the threshold dynamics of F1 and V1 in R1. The parameters $K_F$, $K_{RAD}$, $K_L$, and D were the same for V2 and F2, but, even if they were to vary for V2 to account for the diminished gain above threshold, this should be reflected in a proportionally large increase in the threshold point, which was not observed.

The threshold behaviour of V2 was consistent with additional relaxation channels opening due to the laser emission above the threshold for lasing. Such channels can arise because of reversible photochemistry at the peak emission wavelength between excited state dyes and oxygen, chemical moieties on the surface of M13 or other dyes. In the fundamental rate equations 7 and 8 which describe lasing, the same stimulated emission term $K_{RAD}nD_2$ both feeds the increase in mode occupancy and depletes the population of dyes in the upper state. The additional relaxation channels would break this symmetry by increasing the rate of upper state decay by a factor of $\xi$, which would renormalize the ratio of lasing output to pump energy in the high power limit described by eq. (2) by a factor of $1/\xi$ without affecting the position of the threshold point itself. In the model globally fit to the threshold curves, an asymmetry factor of $\xi = 357\pm37$ for V2 accounts for the flattened response of the output to increased pumping through the threshold point region and the diminished output above threshold, confirming the validity of this theoretical picture. This effect would be enhanced in R2 compared to R1 because the length of the resonant cavity was shortened without changing the length of the gain medium, so the attenuation of the transmission due to absorption channels on the surface of the virus increased by a power of 10.4 compared to R1, which is the ratio of their respective round-trip times.

In the following, the proof of concept mix-and-measure ligand-binding assay is discussed. Almost all assay formats gain sensitivity by trading a degree of disruption to the biological system, for instance, by immobilising the ligands or probes and introducing wash steps, or by labelling the ligands. Given the unprecedented signal-generation capabilities of viral-lasing probes, it was intended to test the feasibility of a mix-and-measure ligand-binding assay which does not make these trade-offs.

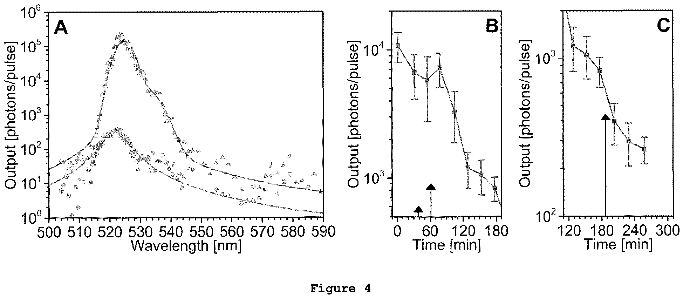

Therefore, the experiment was intended by adding a monoclonal antibody (cp-mAb, illustrated in Fig. 1a) to the reservoir feeding the gain medium, which binds specifically to the five gene 3 coat proteins at one end of the phage, and monitoring the effect on the laser system (Fig. 4).

For both V2 and F2, threshold and spectral measurements were acquired before (Fig. 3) and after (Fig. 4a) the addition of cp-mAb. Between these measurement sets, for V2 only, intensity data was collected at three different levels of pumping 15 min after titrating either a buffer control or cp-mAb into the reservoir containing the detection probes (Fig. 4a, b).

For V2, lasing could no longer be sustained with pump pulses of $1.4 \times 10^{16}$ photons/pulse after the addition of 90 fmol/mL cp-mAb, resulting in a 690-fold decrease in the output at 524.8 nm (Fig. 4a). The 90 fmol/mL cp-mAb was added to the reservoir feeding the gain medium in three steps, so the intensity data reveals the response of the viral laser to these step increases in cp-mAb. The addition of 9 fmol/mL cp-mAb and then another 20 fmol/mL cp-mAb triggered an increase in the threshold point of V2 as the antibody mixed and bound to the surface of the lasing detection probes (Fig. 4b). The threshold point reached a new steady state position after approximately 70 min with no further addition of cp-mAb, but this was then disturbed following the addition of a further 61 fmol/mL cp-mAb, which coincided with the cessation of lasing (Fig. 4c).

The initial 5.5-fold step change at a pump level of $1.3 \times 10^{16}$ photons/pulse was in response to 29 fmol/mL cp-mAb, which equates to 712 lasing detection probes per 1 target biomolecule (Fig. 4b) and when the pumping was below the laser threshold, the addition of cp-mAb had no effect on the output, suggesting that lasing was crucial to the responsivity. The antibodies bound to the phage would red-shift the absorption spectra of certain dyes due to solvent exclusion and contact with a dielectric body with polar side-chains. Theoretical studies of 5 $\mu$m/mL donor dye in bulk solution doped with 0.1 $\mu$m/mL acceptor dye suggest that these red-shifted dyes would sink energy by cavity-enhanced energy transfer and FRET, increasing the threshold point. This effect would be enhanced by homo-FRET transfer between dyes on the same phage, and absorption of the emission by ground state dyes on the antibody-bound phage. This experiment is the first empirical evidence that a bio laser can detect a medically-relevant class of biomolecule and at clinically-relevant concentrations below the probe concentration.

In the control experiment, F2 was only marginally responsive to the addition of 9.1 pmol/mL cp-mAb, showing that fluorescein does not bind cp-mAb and the lasing performance is not inherently affected by the target biomolecule or by any other molecules or salts in the solution (Fig. 3c, d, e). In two further experiments, photo-bleaching did not cause step changes in the threshold point, and neither did the addition of a non-binding antibody. Other explanations for the observed changes in the threshold dynamics, including photo-bleaching, dilution of the probes due to the filter and the addition of buffer, and air bubbles have been ruled out.

Viral lasers are the only category of bio laser that can be genetically programmed to selectively bind a broad range of target biomolecules and chemically modified to deliver a five decade increase in signal-to-noise, 4 nm emission linewidth and large, non-linear responses in output to small changes in probe concentration. The additional optics required for lasing could be readily integrated alongside contemporary optical technologies such as compact pump lasers and photodetectors into existing fluorimeter platforms. Viral lasers can be used to measure clinically-relevant concentrations of biomolecules with a sensitivity approaching that of ELISA, yet without the need for probe immobilisation. This paves the way for genetic reprogramming of viral lasers to selectively bind new target biomolecules directly in solution, which would minimise disruption of the biological system under scrutiny.

The Methods section contains more detail on the sample preparation, the free-space optical assembly, the characterisation of the laser and optical properties, the theoretical models and data analysis.

## Methods

**Sample preparation.** The M13 stock was prepared from a single plaque to ensure genetic homogeneity. M13 was amplified in Top10F' *E. coli* cultures and purified by several rounds of centrifugation with or without polyethylene glycol (PEG-8000) and sodium chloride to either selectively precipitate the phage or to remove cells and cellular debris. The M13 solutions were filter-sterilised with a 0.22 $\mu$m filter. For the experiments performed in the resonator R1, 24.9 mg fluorescein isothiocyanate isomer 1 (FITC) powder was added to 50 mL 2.0 mg/mL M13, 100 mM sodium borate, pH 9.1, and incubated for 2 hr at 37 C prior to quenching with 20 mL 1 M tris-HCl, pH 7.5. For the experiments performed in R2, 15.5 mg FITC powder was added to 29.8 mL 0.64 mg/mL M13, 100 mM sodium borate, pH 9.2, and incubated for 4 hr 15 min at 37 C prior to quenching with 90 mL 133 mM tris-HCl. In both procedures, the dye was removed by rounds of centrifugation with and without PEG-8000/NaCl. The precipitated M13 was resuspended in 100 mM sodium borate, pH 9.0. For the dye-labelled M13 used in the resonator R2, the solution was also driven through a 0.45 $\mu$m filter.

The M13-binding antibody (Life Technologies, M13 phage coat protein monoclonal antibody from clone E1) which recognises the five g3p coat proteins at one end of M13 and the non-binding antibody (Life Technologies, Mouse IgG2a isotype control) were aliquoted and stored at -20 °C at a concentration of 1 mg/mL. Before use, the antibody stock would be thawed prior to dilution to the appropriate concentration. The antibody was added to the reservoir of the gain medium by pipetting the antibody onto the side-wall of the reservoir and washing the side-wall with the sample ejected from the silicone tubing connected to the flow cuvette. The antibody was allowed to mix with the sample solution as it circulated between the reservoir and the flow cuvette prior to measurement for 15 min for V2 (Fig. 4) and for 20 min for F2 (Fig. 3). The buffer control was added in the same way (Fig. 4). The molecular mass of the M13-binding antibody was assumed to be approximately 160 kDa.

**Free-space optical assembly.** The resonant cavities were pumped with pulses of 493 nm light with a FWHM (Full Width at Half Maximum) of 3 ns to 5 ns from an optical parametric oscillator (EKSPLA, NT 342B-20-AW) in single-shot mode via beam-shaping optics consisting of spherical (Comar Optics, 200PB25 and 100NB25) and cylindrical (Comar Optics, 100YB40 and 25UB25) BK7 crown-glass lenses with anti-reflection coatings, which transformed the 4.5 mm diameter circular beam cross-section to a 2 mm x 9 mm ellipse. The pulse intensity was controlled with two Glan-Laser calcite polarizers (Thorlabs, GL10). The first was mounted in a motorised rotation stage (Thorlabs, PRM1/MZ8) connected to a T-cube DC servo controller (Thorlabs, TDC001) and the second was fixed with its polarization axis parallel to the polarization of the excitation pulses. The pump energy was monitored by partially reflecting the light with either, in the resonator R1, a pellicle beamsplitter (Thorlabs, BP208), or, in the resonator R2, a glass window, towards a pyroelectric probe (Molectron, J25) connected to a Joulemeter (Molectron, EM500). In the resonator R1, the detector optics were triggered by the electronic trigger from the pump source; in the resonator R2 they were triggered by an optical trigger (Thorlabs, SM1PD1A) positioned behind a protected aluminum mirror (Thorlabs, PF10-03-G01) in the path between the beamsplitting window and the pyroelectric probe. Additionally, for the resonator R2, the pump was transmitted through a rectangular aperture that matched the dimensions of the cuvette window, and a dielectric shortpass filter with a cut-off wavelength of 510 nm (Comar Optics, 510 IK 50).

The pump impinged on a 2 mm x 8 mm window to a flow cuvette (Starna, 583.2.2FQ-10/Z15) with a 2 mm x 2 mm x 10 mm internal chamber, so the maximum path length of the pump light through the cuvette was 2 mm since the long axis of the internal chamber was orthogonal to the direction of the pump beam and parallel to the axis of the resonant cavity. The samples or cleaning fluids were flowed into the cuvette via silicone tubing from reservoirs using a peristaltic pump (RS Components), and samples would then flow back into the reservoir. The flow rates were 2.5 mL/min for resonator R1 and 3.0 mL/min for R2, and the time between pulses was long enough for the dye in the cuvette to be refreshed. For resonator R2, there was a 5 $\mu$m in-line filter (Whatman, Polydisc HD 5.0 $\mu$m) between the reservoir and the cuvette. In further experiment 2 in resonator R2, the in-line filter was removed and the flow rate was increased to 4 mL/min. The cuvette had 2 mm x 2 mm Spectrosil quartz windows on opposing walls at 90° to the entrance window, which were aligned with the axis of the resonant cavity. The Fresnel reflectance at the glass interface would have been,

$$(3)\, r = \left(\frac{n_1 - n_2}{n_1 + n_2}\right)^2$$

so that **r = 0.0350** at the glass-air interface for **n(fused-silica) = 1.46** and **n(air) = 1.00,** and **r = 0.00217** at the glass-water interface for **n(water) = 1.33.** Consequently, the transmission through the cuvette was nominally 0.860 per round trip of the resonant cavity.

The resonator R1 comprises a flat dielectric output coupler (Comar Optics, 25MX02) and a dielectric spherical mirror with a radius of curvature of 400 mm (Thorlabs, CM508-200-E02), separated by 300 mm with the cuvette adjacent to the output coupler. In the resonator R2, the spherical mirror was replaced with a compound optic consisting of a protected silver spherical mirror with a radius of curvature of 50 mm (Thorlabs, CM254-025-P01) bonded to a 300 $\mu$m pinhole (Thorlabs, P300S) and the separation of the mirrors was reduced to 26 mm. The reflectivity of the flat mirror was 99 % and the reflectivities of the dielectric and metallic spherical mirrors were 99.6 % at 530 nm unpolarized and 98.8 % at 520 nm unpolarized, respectively, based on product data from the manufacturers. The fraction of the oscillation retained is then nominally 0.848 per round trip for resonator R1, and 0.841 per round trip for resonator R2. The round-trip time for light oscillating between the mirrors of resonant cavities was 2.0 ns for resonator R1 and 195 ps for resonator R2, for a cuvette filled with water. The diameters of the mirrors were much greater than the diameters of the cuvette windows and the pinhole, so the Fresnel number, **F**

$$(4)\, F = \frac{a^2}{L\,\lambda}$$

where a is the radius of the aperture and L is the length of the resonant cavity, was approximately $\frac{(1\,\text{mm})^2}{300\,\text{mm} \times 530\,\text{nm}} = 6.3$ for resonator R1 and $\frac{(0.3\,\text{mm})^2}{26\,\text{mm} \times 520\,\text{nm}} = 6.7$ for resonator R2. In both cases, F > 1, so large diffraction losses are not anticipated.

The effective volume of the gain medium is the volume that is pumped which can contribute to the laser emission. For resonator R1, this is the entire volume of the cuvette chamber that is subjected to pumping, $V_{eff}$ = 8mm $\times$ 2 mm $\times$ 2 mm = 32 $\mu$L. For resonator R2, this volume is reduced due to the pinhole before the spherical mirror. The resonator R2 has a half-confocal resonator geometry, so the radius of the beam at the flat mirror is expected to be a factor of

$\sim \dfrac{1}{\sqrt{2}}$ of the radius of the spherical mirror, which is r ~ 150 $\mu$m due to the pinhole. Using similar cones, the effective volume of the gain medium $V_{eff}$ that is pumped is given by,

$$(5)\, V_{eff} = \frac{\pi}{6}\left(\frac{r}{L}\right)^2 \left(\sqrt{2}-1\right)^2 \left\{ \left(\frac{L}{\sqrt{2}-1}+l_2\right)^3 - \left(\frac{L}{\sqrt{2}-1}+l_1\right)^3 \right\}$$

where $l_1$ is the separation of the flat mirror and the closest edge of the pumped volume, and $l_2$ is the separation of the flat mirror and the furthest edge of the pumped volume. For $l_1$ ~ 4.5 mm, $l_2$ ~ 12.5 mm and L ~ 26 mm, $V_{eff}$(R2) ~ 0.365 $\mu$L.

The absorption of the pump by the gain medium depends on the optical path length of the gain medium in the direction of the pump. For resonator R2, the mean optical path length $l_{mean}$ can be estimated by calculating the mean path length across a cylinder with the same length and volume as the effective volume of the gain medium, so

$$(6)\, l_{mean} = \frac{4}{\pi}\sqrt{\frac{V_{eff}}{\pi\,(l_2-l_1)}}$$

For resonator R2, $l_{mean}$ ~ 153 $\mu$m. For resonator R1, $l_{mean}$ = 2 mm, which is the depth of the cuvette chamber.

The light transmitted through the output coupler was collected with either an unoccluded lens (Newport, KPX112) or a lens coupled with an iris diaphragm (Thorlabs, LA1484-A, and Thorlabs, SM1D12CZ) such that the f/numbers were $^{300\ mm}/_{22.9\ mm}$ = 13.1 for resonator R1 and $^{300\ mm}/_{5.5\ mm}$ = 54.5 for resonator R2, respectively. A custom-built spectrometer combining a photomultiplier tube (PMT) (Hamamatsu Photonics, R1166) connected to a high voltage supply (Hamamatsu Photonics, C9525) and a Czerny-Turner monochromator (Bentham, M300EA) with a focal length of 300 mm, f/number of 4.2 and a 1800 lines/mm grating, recorded the output. Before the spectrometer, the output could be optically attenuated using UV fused-silica reflective neutral density filters with ODs ranging from 0.5 to 3.0 for resonator R1 and 0.5 to 4.0 for resonator R2 (Thorlabs) mounted in a filter wheel working in conjunction with a OD 4.0 filter in a flip mount. Optical feedback was prevented by aligning the flip mount for resonator R1 and resonator R2 and the filter wheel for R2 so that the normal to each filter was not parallel to the direction of the incident light. For R1 the mounts were manual but for R2 they were replaced with motorised equivalents (Thorlabs, FW102C, and Thorlabs, MFF102/M). For resonator R2, optomechanics prevented stray light from entering the spectrometer, and a dielectric longpass filter with a 500 nm cut-on (Thorlabs, FEL0500) connected to the light-collecting lens blocked any scattered pump light. For resonator R2, the bias voltage to the PMT was -860 V because this enabled single photon measurements without compromising linearity.

The voltage across a 50 $\Omega$ terminator (for resonator R2, Pico Technology, TA051) connected to the PMT was recorded using an oscilloscope (for resonator R1, Tektronix, TDS210 and for resonator R2, Tektronix, TDS 3052). The waveforms were transferred to a PC for real-time analysis in a custom program that controlled the optical assembly (National Instruments, LabVIEW 8).

The term "pump" refers to the radiant energy input into the resonant cavities in units of photons/pulse, which can be converted to a spectral irradiance of the resonant cavities via multiplication by $\hbar\,\omega$/16 mm$^2$/$\delta\lambda$(0.12 nm)/4 ns pulse, since the area of the cuvette window was 16 mm$^2$, the spectral linewidth of the OPO was 0.12 nm and the optical pulse length was 3 ns to 5 ns, and $\hbar\,\omega$ is the energy of one photon with a wavelength of 493 nm. The term "output" refers to the radiant energy emitted from the resonant cavities in the direction of the detection optics. The reported output is different to the signal observed by the detector by a factor that accounts for the optical attenuation of the spectrometer, and does not account for the radiation that emits in directions that bypass the spectrometer. The oscillation build-up in the resonant cavity would have been greater than the measured output. The output from resonator R2 was calibrated by measuring the response of the detection electronics to single photons, so it is reported in units of photons/pulse. Equivalently, the output from resonator R2 can be converted to a spectral radiance emitted from the resonant cavity via multiplication by $\hbar\,\omega$/1.3 x 10$^{-3}$ sr/$\delta\lambda$(0.4 nm)/pulse, where the solid angle of the spectrometer is 1.3 x 10$^{-3}$ sr and the linewidth of the monochromator is approximately 0.4 nm, and $\hbar\,\omega$ is the energy of one photon at the selected wavelength of the monochromator. Resonator R1 was not calibrated in the same way so it is reported in arbitrary units proportional to the number of photons emitted from the resonant cavity in the direction of the detection optics.

**Characterisation of laser properties.** The threshold dynamics were recorded by measuring the output at a fixed wavelength at variable pump levels. The intensity of the pump pulses were controlled by rotating the first polarizer. If the output was outside of the linear range of the spectrometer, the optical attenuation was adjusted to compensate and the measurement was repeated. In resonator R1, the wavelength was fixed to 527.0 nm and 528.0 nm for the measurements of fluorescein and dye-labelled M13, respectively. In resonator R2, the wavelength was fixed to 520.5 nm and 523.5 nm for the measurements of fluorescein and dye-labelled M13, respectively, except for further experiment 2, for which the wavelength was fixed to 525.0 nm. The spectral measurements were acquired by fixing the pump level and recording the output at different emission wavelengths by rotating the monochromator grating.

**Characterisation of optical properties.** The absorption spectra were recorded using a UV-Vis spectrophotometer (for experiments using resonator R1, Hitachi, Digilab U-1800; for experiments using resonator R2, Thermo Scientific, Nanodrop 2000C) with 1 cm path length cuvettes. The extinction coefficient of M13 is known to be 6.9 x $10^7$ $M^{-1}$ $cm^{-1}$. The absorption of both the M13 and the fluorescein at both the dye peak and at 269 nm were accounted for in the calculations of the concentration of dye-labelled M13 and the number of attached dyes. The precision of the stated concentrations was limited by the precision of the pipettes used (Gilson, Pipetman) and the precision of the UV-Vis spectrophotometers.

For samples measured in experiments in resonator R2, the extinction coefficient of 34.0 $\mu$g/mL fluorescein, 100 mM sodium borate, pH 9.0 was calculated to be 82752 $M^{-1}$ $cm^{-1}$ at the peak absorption at 490 nm and 977 $M^{-1}$ $cm^{-1}$ at 269 nm; the extinction coefficient of M13 at the absorption peak for the attached dyes at 494 nm was 1.5 x $10^6$ $M^{-1}$ $cm^{-1}$. The extinction coefficients of fluorescein attached to M13 were assumed to be the same as for unattached fluorescein at 269 nm and at the absorption peak. The concentration of the dyes attached to the dye-labelled M13 was 12.8 nmol/mL and the concentration of dye-labelled M13 was 20.8 pmol/mL, so there were 618 dyes per M13 on average. The fluorescein was diluted by a factor of 0.13 before further measurements were acquired, so its final concentration was 13.3 nmol/mL.

For sample measured in experiments in resonator R1, the extinction coefficient of 100 $\mu$g/mL fluorescein, 100 mM sodium borate, pH 9.0 was calculated to be 82413 $M^{-1}$ $cm^{-1}$ at the peak absorption at 491 nm and 10634 $M^{-1}$ $cm^{-1}$ at 269 nm; the extinction coefficient of M13 at the absorption peak for the attached dyes at 493 nm was determined to be 1.5 x $10^6$ $M^{-1}$ $cm^{-1}$. The concentration of the dyes attached to the dye-labelled M13 was calculated to be 159 nmol/mL and the initial concentration of dye-labelled M13 was 377 pmol/mL, so there were 422 dyes per M13 on average. The initial concentration of fluorescein was 301 nmol/mL. For both resonator R1 and resonator R2, the wavelength of the pump was 493 nm. For resonator R1, the extinction coefficients at 493 nm compared to the extinction coefficient at the absorption peaks were a factor of 0.976 and 1 for fluorescein and dye-labelled M13, respectively. For resonator R2 these factors were 0.984 and 0.991, respectively. The separation of fluorescein dyes in solution was calculated by taking the cube root of the mean volume per dye. **Theoretical models.** The changes in the energy build-up in a resonator mode and in the upper state population of the dyes can be modelled using the two rate equations:

$$(7)\frac{dn}{dt} = K_{RAD} D_2 (n + n_0) + K_{RAD} D_2 - K_L n$$

$$(8)\frac{dD_2}{dt} = K_F \varphi (D - D_2) - K_{RAD} D_2 (n + n_0) \xi - K_2 D_2$$

where n is the energy in the resonator mode, $\varphi$ is the rate of pumping of the resonator mode, D is the number of dyes in the resonator mode volume, $D_1$ and $D_2$ are the number of dyes in the lower and upper state of the laser transition, respectively, $K_F$ is the strength of the coupling of the pump with the dyes, $K_{RAD}$ is the rate of emission into the mode per dye, $K_2$ is the rate of decay from the upper state per dye, $K_L$ is the rate of loss from the resonator, $n_0$ is the seed fluorescence and $\xi$ is the asymmetry factor which is typically equal to 1.

In eq. (7), the first and second term describe the rate of stimulated and spontaneous emission, and the third term describes the rate of loss from the resonator. In eq. (8), the first term describes the rate of pumping of the dyes, and the second and third terms describe the depopulation of the upper state due to stimulated emission and all other sources, respectively.

The rate of pumping is derived from the Beer-Lambert law assuming that every pump photon absorbed results in a dye in the upper state:

$$(9)\frac{dD_2}{dt} = \varphi \left(1 - e^{-\frac{D_1}{v} \varepsilon l}\right) \sim \varphi D_1 \frac{\varepsilon l}{v} = K_F \varphi (D - D_2)$$

where $\varepsilon$ is the extinction coefficient, **l** is the optical depth of the resonator mode and **v** is the volume of the resonator mode that is being pumped. This approximation is valid for resonator R2 because the concentration of the dyes is low and the path length across the resonant cavity mode is narrow. For resonator R1, these assumptions do not strictly hold, but are necessary to simplify the equations so that they can be solved analytically. Additionally, the Beer-Lambert law assumes that the dyes are homogeneously distributed in the solution, which is not the case for gain media composed of dye-labelled M13.

The rate of radiative emission per dye into the resonator mode is a function of the rate of spontaneous emission and the number of resonator modes:

$$(10) \qquad K_{RAD} = \frac{\gamma_{RAD}}{\rho}$$

where $\gamma_{RAD}$ is the rate of spontaneous emission per dye and $\rho$ is the number **of** resonator modes.

The rate of decay from the upper state per dye is the sum of the rate of spontaneous emission and the rate of non-radiative decay:

$$(11) \quad K_2 = \gamma_{RAD} + \gamma_{NON}$$

where $\gamma_{NON}$ is the rate of non-radiative decay per dye.

$K_L$ is determined by the loss rate of the resonator, which is given by

$$(12) \quad K_L = -\frac{1}{\tau_r} \ln(1 - LOSS)$$

where $\tau_r$ is the round-trip time and LOSS is the fraction of the oscillation build-up lost per round-trip.

The energy in the resonator mode is derived from eq. (7) and eq. (8) by assuming steady state emission, eliminating $D_2$ and solving the resulting quadratic equation:

$$(13) \quad n =
\frac{\beta}{2\,\xi\,K_{RAD}} \left\{ K_F\,\varphi \left( \frac{D\,K_{RAD}}{K_L} - 1 \right) - \xi\,K_{RAD}\,n_0 - K_2 + \sqrt{\left( K_F\,\varphi \left( \frac{D\,K_{RAD}}{K_L} - 1 \right) - \xi\,K_{RAD}\,n_0 - K_2 \right)^2 + \frac{4\,K_F\,\varphi\,D\,\xi\,K_{RAD}^2}{K_L}(n_0 + 1)} \right\}$$

where $\beta$ is the optical attenuation due to the output coupler and the spectral bandwidth of the spectrometer.

The build-up of energy in the dominant resonator mode depends on optical amplification, which implies the presence of a non-zero seed fluorescence $n_0$ prior to amplification. This is modelled as the energy in the resonator mode when there is no stimulated emission. From eq. (7) and eq. (8):

$$(14) \quad \frac{dn_0}{dt} = \theta\,\gamma_{RAD}\,D_2 - K_L\,n_0$$

$$(15) \quad \frac{dD_2}{dt} = K_F\,\varphi\,(D - D_2) - K_2\,D_2$$

where $\theta$ is the fraction of the spontaneous emission that emits at a wavelength and in a direction such that it can contribute to the seed fluorescence. Using the same assumptions as for eq. (13):

$$(16) \quad n_0 = \frac{D\,\theta\,\gamma_{RAD}}{K_L \left( 1 + \frac{K_2}{K_F\,\varphi} \right)}$$

There is a background fluorescence from dyes that are pumped but are not contained within the resonator mode volume. As for the seed fluorescence, there is no stimulated emission, so eq. (7) and eq. (8) are reduced to:

$$(17) \quad \frac{dn}{dt} = \mu \, \gamma_{RAD} \, D_2' - K_L' \, n$$

$$(18) \quad \frac{dD_2'}{dt} = K_F' \, \varphi' \, (D' - D_2') - K_2' \, D_2'$$

where the apostrophe indicates that the variable or parameter is different for the dyes outside of the resonator mode volume compared to the dyes inside of this volume. For instance, for resonator R2 the volume of the cuvette chamber that overlaps the resonator mode volume is a small fraction of the total cuvette chamber volume, so D' ≫ D. Likewise, the fraction of the pump $\varphi$ that impinges on the mode volume is small. The change in volume also accounts for the change in $K_F$, see eq. (9).

There is no optical feedback so the background fluorescence is lost from the resonator at the rate $K_L'$ which light can travel out of the resonator. In this model, $K_2$ is allowed to differ from $K_2'$ because the rate of non-radiative decay may be different for dyes inside and outside of the resonator mode since there are more channels for interaction between dyes inside the resonator mode. $\mu$ is the fraction of the spontaneous emission from dyes outside of the resonator mode volume that enters the spectrometer, which is dependent on the solid angle subtended by the light-collecting lens.

Using the same assumptions as for eq. (13) and eq. (16), the background fluorescence is given by

$$(19) \quad n = \frac{D' \, \mu \, \gamma_{RAD}}{K_L' \left(1 + \frac{K_2'}{K_F' \, \varphi'}\right)}$$

Light may have entered the spectrometer from external sources to the resonant cavity, which is accounted for by a constant offset.

The threshold point $\varphi_{th}$ is given by

$$(20) \quad K_F \, \varphi_{th} \left(\frac{D \, K_{RAD}}{K_L} - 1\right) - \xi \, K_{RAD} \, n_0 - K_2 = 0$$

from eq. (13). Including the term for the seed fluorescence, this is then

$$(21) \quad K_F \, \varphi_{th} \left(\frac{D \, K_{RAD}}{K_L} - 1\right) - \xi \, K_{RAD} \, \frac{D \, \theta \, \gamma_{RAD}}{K_L \left(1 + \frac{K_2}{K_F \, \varphi_{th}}\right)} - K_2 = 0$$

which can be solved to find

$$(22) \quad \varphi_{th} = \frac{K_2 \, (2 \, K_L - D \, K_{RAD}) + D \, K_{RAD} \left\{ \theta \, \gamma_{RAD} \, \xi + \sqrt{(\theta \, \gamma_{RAD} \, \xi - K_2)^2 + \frac{4 \, \theta \, \gamma_{RAD} \, \xi \, K_2 \, K_L}{D \, K_{RAD}}} \right\}}{2 \, K_F \, (D \, K_{RAD} - K_L)}$$

From eq. (11), this can be simplified by noting that

$$(23) \quad \theta \, \gamma_{RAD} \, \xi \ll K_2 = \gamma_{RAD} + \gamma_{NON}$$

since

$$(24) \quad \theta \, \xi \ll 1$$

and that for gain to exceed losses

$$(25) \quad \frac{D\, K_{RAD}}{K_L} > 1$$

so that the threshold point is given by

$$(1) \quad \varphi_{th} = \frac{K_2\, K_L}{K_F\, (D\, K_{RAD} - K_L)}$$

The minimum number of dyes required to achieve threshold $D_{min}$ even at very large levels of pumping, is given by,

$$(26) \quad D_{MIN} = \frac{K_L}{K_{RAD}}$$

The output far above threshold when $\varphi \to \infty$ is given by

$$(27) \quad \frac{n}{\varphi} \to \frac{\beta\, K_F}{\xi\, K_{RAD}} \left( \frac{D\, K_{RAD}}{K_L} - 1 \right)$$

If there is no gain depletion above threshold, then $\xi = 1$,

$$(2) \quad \frac{n}{\varphi} \to \frac{\beta\, K_F}{K_{RAD}} \left( \frac{D\, K_{RAD}}{K_L} - 1 \right)$$

Furthermore, if $D \gg D_{min}$ then

$$(28) \quad \frac{n}{\varphi} \to \frac{\beta\, K_F\, D}{K_L}$$

and the gain above threshold ceases to depend on the rate of spontaneous emission.

The time-dependence of the pump energy was accounted for by assuming a Gaussian profile with a FWHM of 4 ns,

$$(29) \quad \varphi(t) = \varphi_0 \times Gaussian(t)$$

and calculating the output based on the peak pump energy. The non-linear response of output to increased pumping means that the peak pump energy has the greatest contribution to the overall output. An alternative method was tested in which the pump pulse was split into sub-nanosecond time bins over a $\sim 50$ ns time window centred about the pulse peak and the output calculated by summing the outputs from each time bin, but the results generated were negligibly different to the preferred method. The calculation of the threshold point accounted for the time-dependence of the pump energy.

For R2, only $\frac{1}{2\,mm}$ of the pump impinged on the resonator mode because its diameter was less than the 2 mm height of the window. The remainder would have pumped dyes outside of the dominant resonator mode.

The threshold point for each set of data for a different concentration of dye-labelled M13 or fluorescein could be readily determined using a model similar to eq. (13),

$$(30) \quad n = \chi_0 + \chi_1 \left\{ \left( \frac{\varphi}{\varphi_{th}} - 1 \right) + \sqrt{ \left( \frac{\varphi}{\varphi_{th}} - 1 \right)^2 + \chi_2\, \varphi } \right\}$$

where $\chi_n$ are fit parameters that are not directly linked to the experimentally observable variables, $\varphi_{th}$ is the threshold point and the time-dependence of the pump is disregarded.

**Data analysis.** For resonator R1, the threshold dynamics of fluorescein and dye-labelled M13 were analysed by globally fitting each set of data with a model that employed eq. (13) as well as eq. (16) for the seed fluorescence. The parameters $\gamma_{RAD}$, $K_L$, $K_F$, $I_{mean}$, $V_{eff}$ and $\xi$ were all fixed and the same for each set of measurements. Each of these were calculated in advance (see Free-space optical assembly and Theoretical Models), except for $\gamma_{RAD}$ which is known to equal 0.25 ns$^{-1}$.

D was fixed but different for each set of measurements. A factor was calculated from the spectral measurements to account for the difference between the output at the measured emission wavelength and the spectral peak, which was fixed for each set of measurements except for 146 nmol/mL fluorescein, since there was no spectral measurement of this sample and it may have undergone a spectral shift. The parameters $\rho$, $\beta$, $\theta$ and a constant offset to account for external light sources were not fixed and were shared between each set of data because they should be consistent between measurements. The parameter $\gamma_{NON}$ was not fixed and shared between the sets of dye-labelled M13 measurements, but allowed to vary individually for each set of fluorescein measurements.

In Fig. 2c, the line representing the threshold point as a function of probe concentration was simulated using this model. In Fig. 2d, the pump energy values were selected because they represent local minima in a plot of pulse energy variability against pulse energy. The pulse energy varies from pulse to pulse due to variability in the intensity of the emission from the OPO. The lines were simulated using the same model.

The threshold dynamics of fluorescein and dye-labelled M13 in resonator R2 were analysed using a similar model to resonator R1. Since the effective mode volume was smaller, eq. (19) was employed to account for the background fluorescence from dyes outside of the mode volume. The additional parameters $K'_L$ and $K'_F$ were fixed and the same for each set of measurements, and D' was fixed but different for each set of measurements. $\gamma'_{NON}$ was fixed at zero for each set of measurements and the additional parameter $\mu$ was not fixed but shared between each set of measurements. The parameter $\gamma_{NON}$ was not fixed and allowed to vary individually for each set of measurements, except for the fluorescein measurement that did not contain cp-mAb for which it was fixed to zero. The parameter $\xi$ was not fixed and shared between the dye-labelled M13 measurements, but fixed to 1 for the fluorescein measurements. The reported error on $\xi$ is the standard error. The threshold dynamics of fluorescein and the dye-labelled M13 as measured in the two further experiments were analysed in the same way, except that $\xi$ was shared between dye-labelled M13 measurements from the same experiment only.

For experiments conducted in resonator R1, the threshold measurements were additionally fit using eq. (30) to accurately determine the position of the threshold point. From eq. (1), as $D \rightarrow D_{MIN}$, $\varphi_{th} \rightarrow \infty$ and if $D < D_{MIN}$, then $\varphi_{th} < 0$, so the parameter $\varphi_{th}$ was substituted for its reciprocal in the fitting function and subsequently derived after the model converged. The error bars are standard errors. The error bar in Fig. 2c that extends beyond the upper limit of the y-axis extends to infinity, and $\varphi_{th} < 0$ for the most dilute dye-labelled M13 sample, implying that the probe concentration was insufficient to achieve lasing.

For experiments conducted in resonator R1, when the pumping was above the threshold for lasing the spectra were fit to a model consisting of a sum of two Gaussian functions with a constant offset. For experiments conducted in resonator R2, the above-threshold spectra were fit to a model consisting of a single Voigt function or a sum of two Voigt functions with no constant offset, and the below-threshold spectra were fit to a Lorentzian function with no constant offset. In Fig. 3, the scatter points in the spectra are the means of four measurements at each wavelength. The mean and coefficient of variation of the pump energy has been reported for each emission spectrum. The spectral peak centres and linewidths were determined by the model fitting and the reported errors are standard errors.

## Description of further experiments in resonator R2

Two additional experiments were performed in resonator R2 using the same dye-labelled M13 sample to test whether photobleaching could cause the step changes in the output. Experiment 1 was similar to the experiment described previously, except that 91 fmol/mL mouse IgG2a isotype control which should not bind M13 was added to the reservoir before 91 fmol/mL cp mAb was added, which was then increased to 1.9 pmol/mL. Buffer was not added to the reservoir between measurements and the antibody was allowed mix for at least 12 min prior to measurement. Experiment 2 was similar to the previous experiments, except that the dye-labelled M13 was diluted by a factor of ~ 0.89 in type 1 water and the final sample volume was ~ 2.75 mL, which was less than the 6.85 mL used in previous experiments, and the in-line filter was removed. Also, prolonged storage of the dye labelled M13 at 4 °C resulted in some sedimentation, so the solution was transferred to a fresh container and then centrifuged to remove any residual insoluble material. For this experiment, only 4.5 pmol/mL cp mAb was added. The changes in the threshold dynamics with time before and after the addition of antibody were measured in both experiments. Emission spectra before and after the addition of antibody were acquired for the second experiment but not for the first experiment.

**Extended technical description of proof of concept mix and measure ligand binding assay**

Other experimental factors might have resulted in a decrease in the viral laser output, and we consider three possibilities here. The first and most trivial is the dilution of the dye with the addition of antibody solution. This is ruled out on arithmetic grounds: the 5 $\mu$m pore size of the filter was much larger than M13 and the addition of 100 $\mu$L buffer to the 6.85 mL reservoir would not have had a profound effect unless the initial concentration of probes was less than 1.5 % above the minimum required to achieve lasing. The second is air bubbles in the cuvette chamber. Whilst air did sometimes pass through the flow cuvette between measurements, the system was designed to allow air to escape and the variation of the output did not increase as the mean decreased, which would indicate a trapped bubble partially occluding the resonant cavity. Third, because of the repetitive nature of the experiment, photobleaching effects could produce reductions in output which might be confused with those due to the binding antibody. For instance, photobleaching of the dyes most likely caused the decrease in the output below threshold and the output above threshold between the spectral measurement, threshold measurement and before adding cp-mAb in the titration measurement. However, photobleaching does not account for the step changes in the output during the titration measurement: there was no decrease below threshold and there were only 231 recorded excitation pulses compared to 1200 recorded pulses for the preceding spectral and threshold measurements. In two further experiments, photobleaching did not cause step changes in the threshold point, and neither did the addition of a non-binding antibody. In one control experiment, the addition of cp-mAb led to a sharp increase in the threshold point, and in another the results were ambiguous due to greater noise levels.

**Optical configuration diagrams**

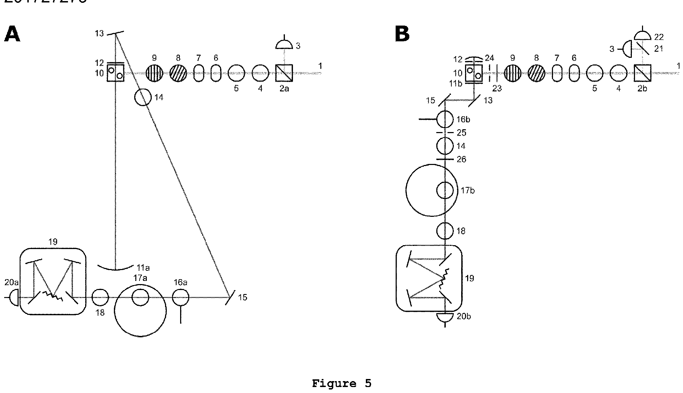

Figure 5 shows the optical configuration for experiments conducted using **a,** resonant cavity R1 and **b,** resonant cavity R2. The numerical labels in **a** and **b** refer to the following list of components:

1 - Optical parametric oscillator via periscope with two flat 1" Ag mirrors
2a - Pellicle beamsplitter
2b - Beamsplitting window
3 - Beam monitor: pyroelectric probe connected to a Joulemeter
4, 5 - Spherical beam-expanding telescope
6, 7 - Cylindrical beam-reducing telescope
8, 9 - Pump energy control: two Glan-Laser calcite polarizers, 8 in a motorised rotation stage, 9 fixed in a manual rotation stage
10 - Flow cuvette with 40 $\mu$l chamber
11a - Spherical dielectric mirror, R = 400 mm
11b - 300 $\mu$m pinhole bonded to a spherical Ag mirror, R = 50 mm
12 - Flat, dielectric output coupler
13 - Flat 1" Ag mirror
14 - 1" spherical convex lens
15 - Flat 1" Ag mirror
16a - OD 4 reflective ND filter in manual flipper mount
16b - OD 4 reflective ND filter in motorised flipper mount
17a - Manual filter wheel mounted with OD 0.5, 1.0, 2.0, 3.0 reflective ND filters, and one unmounted position
17b - Motorised filter wheel mounted with OD 0.5, 1.0, 2.0, 3.0, 4.0 reflective ND filters, and one unmounted position
18 - Spherical convex lens
19 - Monochromator with a 1200 grooves/mm grating
20a - Detector: PMT connected to a 50 $\Omega$ terminator, voltage measured by a 60 MHz oscilloscope
20b - Same as 20a, except with a 500 MHz oscilloscope and a 1 GHz feed-through 50 $\Omega$ terminator
21 - Beamsplitting flat 1" Al mirror
22 - Optical trigger to oscilloscope: Si photodiode connected to high-speed circuit
23 - Shortpass filter, $\lambda$ = 510 nm.
24 - Rectangluar aperture
25 - Iris diaphragm
26 - Longpass filter, $\lambda$ = 500 nm

**Claims**

1.  A method for bio-sensing a binding ability among a biomolecule, such as an antibody, a membrane protein or the like, and a virus, comprising the steps of:

    a) labelling the virus by conjugating a plurality of dyes, preferably fluorescein dyes, to the virus in order to achieve a labelled virus being controllable with respect to the density of labelled dyes per virus molecule;
    b) binding the labelled virus to the biomolecule in a liquid solution to the extent the binding characteristics among the biomolecule and the labelled virus allow for in a ligand binding assay in order generate biomolecule virus compounds;
    c) pumping the liquid solution comprising the biomolecule virus compound with light having a wavelength and/or an intensity being adopted to the type of the effective mechanism of the dyes, preferably the effective fluorescence mechanism of the fluorescein dyes, labelled to the virus;
    d) amplifying the light emitted from the pumped dyes in an optical resonator; and
    e) measuring the intensity of the amplified emitted light and evaluating the intensity of the amplified emitted light against the known density of labelled fluorescein dyes per virus molecule or against an equivalent concentration of the same number of dyes per probe in order determine the binding ability of the biomolecule to the labelled virus.

2.  The method according to claim 1 wherein the virus is a M13 bacteriophage or a recombinant Tobacco mosaic virus-like particle (rTMV).

3.  The method according to any of the preceding claims, wherein the biomolecule an antibody or a monoclonal antibody, preferably an IgG2a monoclonal antibody (mAB).

4.  The method according to any of the preceding claims wherein the fluorescein dye is a fluorescein isothiocyanate isomer 1 dye.

5.  The method according to any of the preceding claims wherein the virus is covalently modified with the fluorescein dye.

6.  The method according to any of the preceding claims wherein the pumping is achieved using 1 ns to 20 ns excitation pulses in the wavelength of the visible light, preferably in the range of 450 to 600 nm.

7.  The method according to any of the preceding claims wherein the liquid solution is circulated between a reservoir and a flow cuvette.

8.  The method according to any of the preceding claims wherein the virus is effectively genetically-programmable with a spectral peak emission that is tunable by varying the number of fluorescein dyes attached per virus and/or by modifying the chemical landscape of the surface of the virus.

9.  The method according to claim 8, wherein the virus is conjugated with a number of fluorescein dyes equivalent to a range of 0.5 to 2 dyes, preferably 0.8 to 1.2 dyes, on average per ring of coat proteins of the virus.

**A** FITC labelled M13 phage

Target biomolecule:
monoclonal antibody

2.8 nm
0.9 nm
3.3 nm

Chromophore

Linker

FITC

**B** PD

Pyrometer

Resonator
Polarizers

Steering
mirrors
Filters
Beam-shaping
lenses
from
OPO

Iris
ND filter in flipper
mount

Collimating lens

Longpass
filter
ND filter
wheel

Focusing lens

Monochromator

PMT

Figure 1

Figure 2

Figure 3

Figure 4

Figure 5

Europäisches
Patentamt

European
Patent Office

Office européen
des brevets

# EUROPEAN SEARCH REPORT

Application Number

EP 18 19 5469

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Hales J E: "Implementing the synthetic biology design cycle to fabricate laser dyes", <br><br> , <br> 31 December 2014 (2014-12-31), <br> XP002787925, <br> Retrieved from the Internet: <br> URL:http://iris.ucl.ac.uk/iris/publication/955819/1 <br> [retrieved on 2019-01-14] <br> * abstract * <br> ----- | 1-9 | INV. <br> G01N33/52 |
| X <br> Y | EP 2 794 861 A1 (UCL BUSINESS PLC [GB]) 29 October 2014 (2014-10-29) <br> * paragraph [0096]; claim 4; figure 10; examples 6-8 * <br> * paragraph [0055] - paragraph [0058] * <br> * paragraph [0104] * <br> ----- | 1-9 <br> 1-9 | |
| Y | XUDONG FAN ET AL: "The potential of optofluidic biolasers", <br> NATURE METHODS, <br> vol. 11, no. 2, <br> 1 February 2014 (2014-02-01), pages 141-147, XP055541496, <br> New York <br> ISSN: 1548-7091, DOI: 10.1038/nmeth.2805 <br> * abstract; figure 1 * <br> ----- | 1-9 | |

TECHNICAL FIELDS
SEARCHED     (IPC)

G01N

The present search report has been drawn up for all claims

| Place of search | Date of completion of the search | Examiner |
|---|---|---|
| Munich | 14 January 2019 | Hinchliffe, Philippe |

EPO FORM 1503 03.82 (P04C01)

22

**EP 3 502 694 A1**

## ANNEX TO THE EUROPEAN SEARCH REPORT
## ON EUROPEAN PATENT APPLICATION NO.

EP 18 19 5469

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2019

| Patent document cited in search report | | Publication date | Patent family member(s) | | Publication date |
|---|---|---|---|---|---|
| EP 2794861 | A1 | 29-10-2014 | EP | 2794861 A1 | 29-10-2014 |
| | | | US | 2014371106 A1 | 18-12-2014 |
| | | | WO | 2013093499 A1 | 27-06-2013 |

EPO FORM P0459

For more details about this annex : see Official Journal of the European Patent Office, No. 12/82